# EUROPEAN PATENT SPECIFICATION

(11) **EP 2 377 560 B1**
(45) Date of publication and mention of the grant of the patent: **09.01.2013**
(21) Application number: 11158019.7
(22) Date of filing: 14.03.2011
(51) Int. Cl.: A61L 2/04, A61L 2/22, A23L 3/14, B01J 3/04

(54) **Rotating basket sterilizer with water spray nozzles**
Sterilisator mit Drehkorb mit Wassersprühdüsen
Stérilisateur à panier rotatif avec buses de pulvérisation d'eau

(30) Priority: 09.04.2010 IT PR20100024
(43) Date of publication of application: 19.10.2011
(73) Proprietor: Gea Levati Food Tech S.r.l., 43044 Collecchio (Parma) (IT)
(72) Inventor: Donini, Andrea, 43044, COLLECCHIO (PARMA) (IT)
(74) Representative: Gotra, Stefano

(56) References cited:
- EP-A2- 1 862 082
- WO-A1-2005/113024
- US-A- 5 849 246
- US-A1- 2005 226 094

## Description

The present invention refers to the field of the rotating discontinuous sterilizers (known as autoclaves), of the horizontal End-Over-End type having the backpressure sterilization technology.

The main object of the backpressure technology consists of neutralizing the effects of the overpressure which necessarily builds up inside the containers, during the sterilization with flows in the same direction.

As it is known, for performing the sterilization of packaged products, stationary or rotating discontinuous pressurized plants (autoclaves) are used. The rotating-type autoclaves can also be classified in two main groups: the drum autoclaves and the basket autoclaves. The rotating-type basket autoclaves substantially comprise a shell structure whose main chamber receives a rotating frame which in turn rotates a group of rotating baskets receiving the containers containing the product. In said autoclaves, the sterilization is performed with steam or hot water introduced from the upper part of the chamber and distributed on the rotating baskets by a stationary nozzle system or by nozzles integrally rotating with the baskets.

Unfortunately, for obtaining an homogeneous distribution of the temperature in the prior art autoclaves, it is known the use of nozzles integrally rotating with the rotating baskets, the water or steam exiting the nozzles hit the baskets in predetermined points or there are several differently slanted stationary nozzles.

It is known, for example, from patent JP2001231521 a sterilization performed by a nozzle system integrally rotating with the baskets receiving the containers with the product. Another such drum steriliser is known from US-A-2005/226094.

The object of the present invention consists of improving the heat exchange efficiency between the product and the process fluids and of equalizing the sterilization times between all the containers irrespective of their position inside the basket in comparison with the prior art systems, in this way it is obtained a reduction of the sterilization times and consequently an improvement of the quality of the obtained product, by implementing a combined sterilization plant.

Said sterilizer therefore provides both the use of stationary nozzles (located in the upper zone of the sterilization chamber) and of movable nozzles integrally rotating with the baskets.

A first advantage, thanks to the simultaneous use of vertical rain water baskets and rotating nozzles, wherein the water jet slants continuously change and said jets cross the stationary vertical flows of the stationary nozzles, is due to the generation of a water swirl which, besides more easily penetrating in the center of the basket (which is the most difficult location to reach), promotes the heat exchange between water (process fluid) and the product inside the containers.

A second advantage is due to the fact that this type of sterilizer is adapted to any type of container, such as metal boxes, glass jars, envelopes, bowls and beverage paperboards of the Tetra Brik type (trademark registered by AB TETRA PAK, Sweden).

It is possible to appreciate, from what will be described in the following, that another advantage is due to the fact that the quick closure system of this sterilizer ensures an uniform and constant pressure along all the circumference of the door without slidings among the rings.

An advantage is due to the reduced size of the unused areas thanks to the maximum size of the baskets in relation to the inner diameter of the cylindrical vessel.

A further advantage is that the times necessary for introducing and extracting the baskets in/from the sterilization chamber are reduced, said introduction and extraction are performed by an always-in-phase engagement/disengagement automatic system.

Said objects and advantages are all met by a rain water cycle rotating basket sterilizer, object of the present invention, which is characterized by the attached claims.

This and other characteristics will be better understood by the following description of a preferred embodiment shown, in an exemplifying and non limiting way, in the following drawings.
- Figure 1 is a general side view of the sterilizer;
- Figure 2 is a longitudinal cross-section of the sterilizer;

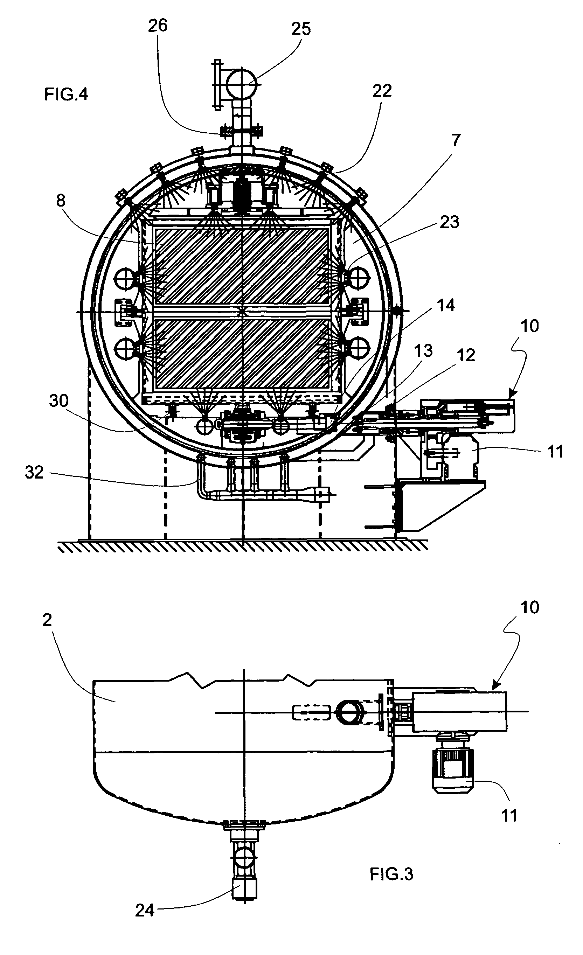

- Figure 3 is a top view of the back portion of the device;
- Figure 4 is an elevation view of a cross-section of the preceding view;

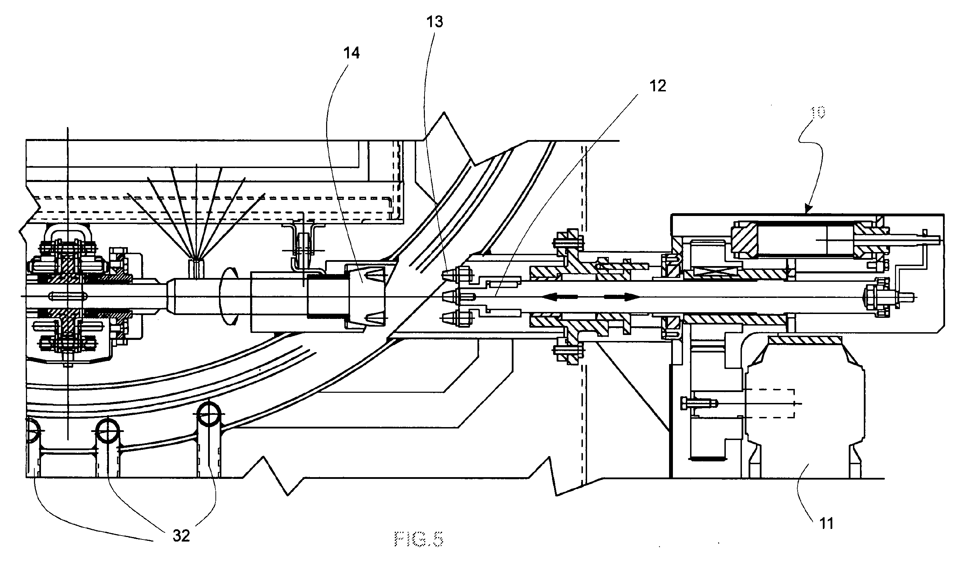

- Figure 5 shows the engagement device with a detail of the centering system;
- Figure 6 is a view of the device particularly showing the driving system of the basket holder frame;
- Figure 7 shows two successive positions of the basket holder frame, the baskets themselves and the movable nozzles integral to the latter.

Referring particularly to the figures, 1 generally shows a rain water cycle rotating basket sterilizer according to the present invention.

Said sterilizer 1 generally comprises a pressurized horizontal cylindrical vessel 2, commonly known as autoclave, having at least one door 3 for introducing and removing the load to be sterilized with a quick closure system 4 made of three ring segments 5 hinged one to the other and driven by a pneumatic actuator 6 located on the upper part of the horizontal cylindrical vessel 2.

The horizontal cylindrical vessel 2 receives a rotating frame 7 which in turn rotates the baskets 8 housing the containers with the product to be sterilized.

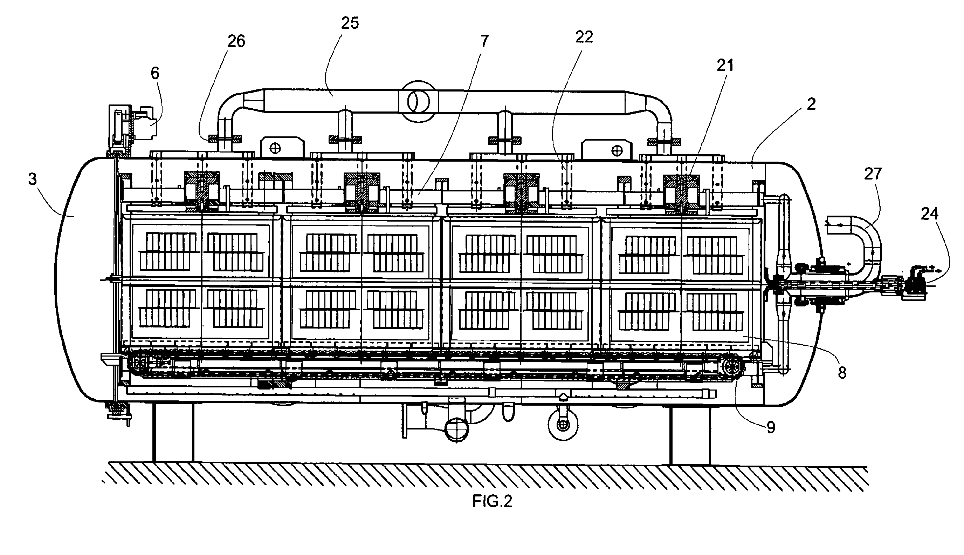

Since baskets 8 receiving the containers layered inside their volume are provided by lower small wheels 30, they are inserted and extracted by a chain conveyor 9 (Figure 2) driven by an always-in-phase automatic engagement/disengagement group 10, driven from the outside by a ratiomotor 11.

For compensating the clearances which there are inevitably in the chain transmission conveyor 9, said group 10 also acts as a centering system because it has a coupling 12 substantially always in phase, formed by four conical pins 13 compensating, at the engagement, the position variable of an opposed halfcoupling 14, due to the clearance of the conveyor 9 chain.

The basket holder frame 7 is supported by four idle wheels 15 received in cylindrical cavities 16 present in the horizontal cylindrical container 2, accessible from the outside and provided with ball bearings 17 having a permanent lubrication and draining into the outer environment to avoid any kind of pollution of the process water. These groups are received in cylindrical supports 18 easily accessible from the outside.

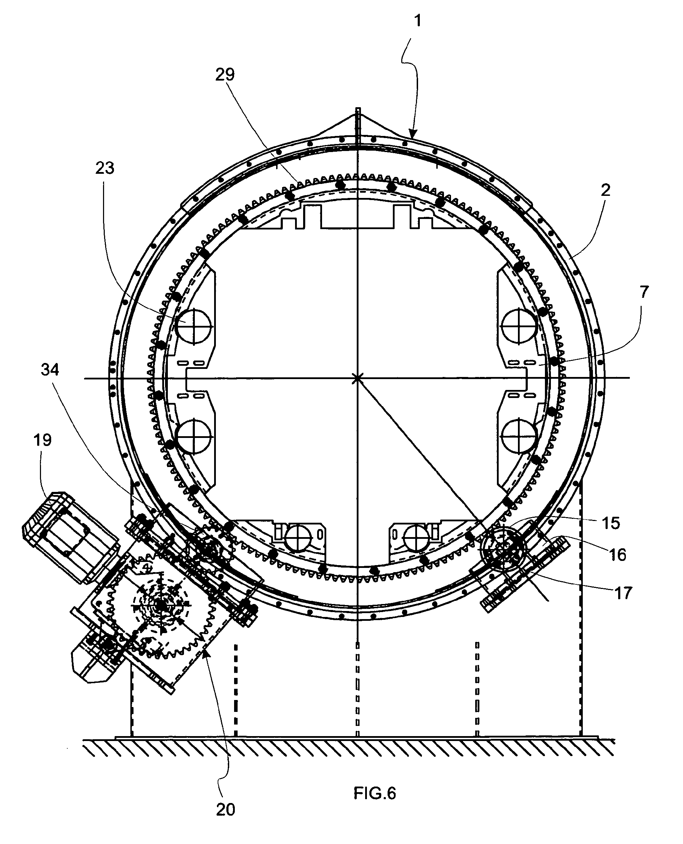

As shown in Figure 6, there are means for adjusting the basket 8 rotation speed, comprising a ratiomotor 19 driven by an inverter. The motor 19 shaft rotates a gear train 20 which, by a pinion 34, transmits the motion to a ring gear 29 to which is fixed the frame 7 and consequently to the baskets 8 receiving the containers. The ratiomotor is driven in order to choose between different rotations: an oscillation, a complete rotation, a clockwise and counter clockwise alternate complete rotations, speed variations, stop in a position for a predetermined time.

A system of pressing pneumatic elements 21, independent for each basket 8 with an outer drive, exerts a constant force on said baskets 8 during the sterilization cycle.

Stationary nozzles 22 spray the process fluid (water according to the preferred embodiment shown in the attached figures), they are preferably located on the upper portion of the horizontal cylindrical vessel 2, wherein their effectiveness is the highest.

Movable nozzles 23 are distributed and fixed to the basket 8 walls (said nozzles are movable with respect to the horizontal cylindrical vessel 2) and the nozzles integrally rotate with said baskets 8. So, during the basket 8 rotation, the water jets exiting the movable nozzles 23 continuously hit a series of predetermined points on the containers receiving the product to be sterilized.

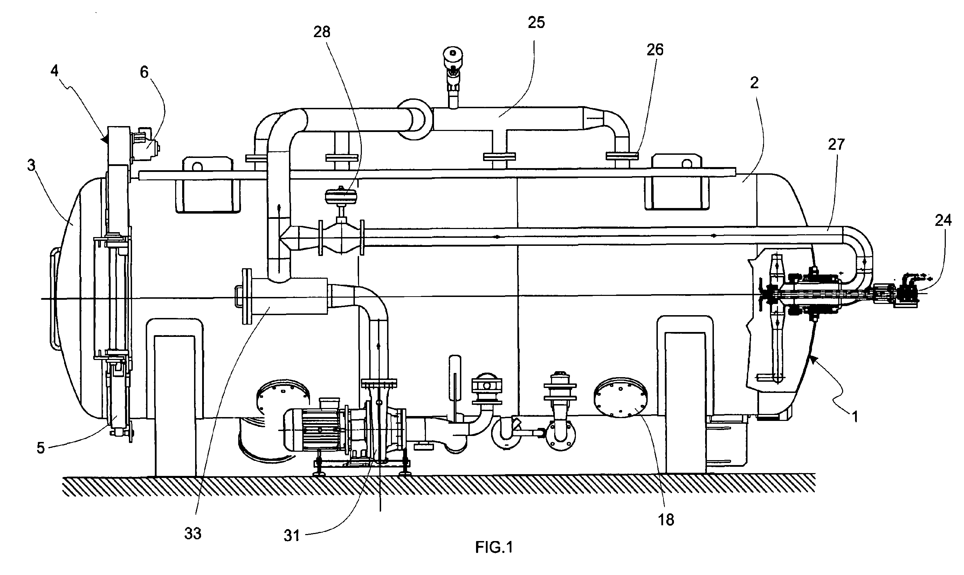

A rotating coupling 24, located in the back portion of the device (Figures 1 and 2), enables the air to enter and exit the pressing elements 21 and the process fluid to enter the movable nozzles 23.

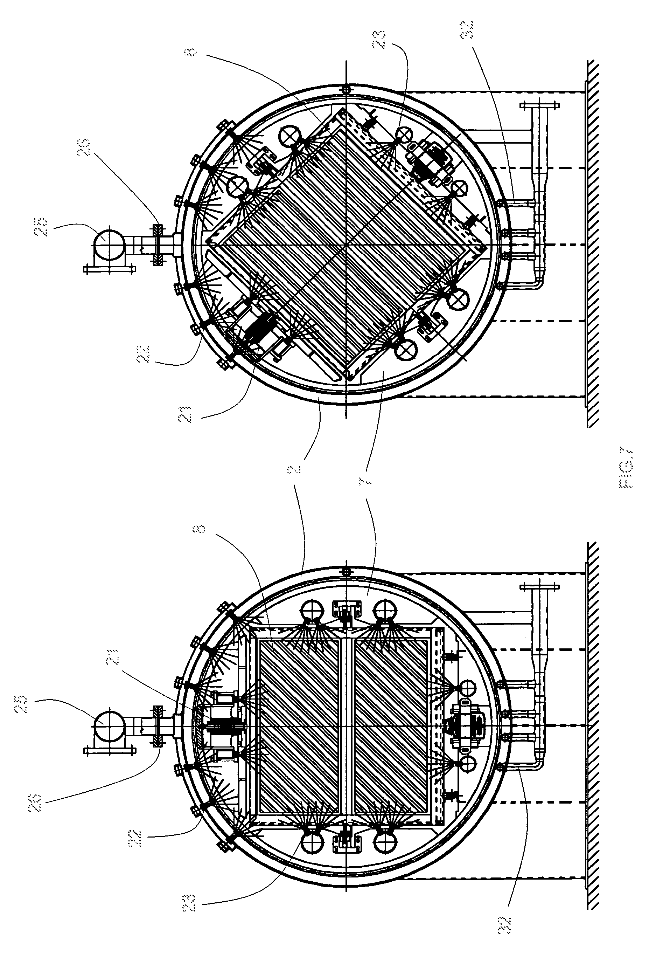

The combination of the basket 8 rotation, of the vertical water jets from the stationary nozzles 22 and of the water jets, which continuously change, from the movable nozzles 23 (Figure 7), forms a water swirl which, besides more easily penetrating the center of the baskets 8, promotes the heat exchange between the water and the product received in the containers.

As it is clearly shown in Figure 1, each stationary nozzle 22 is fed by a main duct 25, to which is connected by flanges 26, in turn fed by a centrifugal water-circulation pump 31.

A pipe 27 comes out said main duct 25, immediately after a filter 33 of the centrifugal pump 31, said pipe 27 transports the process fluid to the movable nozzles 23. There are provided means for adjusting the pressure and the flow rate of the process fluid, in fact there is a modulating valve 28 on the pipe 27 which enables to change the pressure and flow rate of the process fluid for every kind of product, recipe for preparing the product and container to be sterilized.

Consequently, by combining the rotation variables of the baskets 8 and the pressure and flow rate of the water, for every kind of production, it is possible to reduce at the minimum and store the sterilization times with all the obtainable advantages, such as, for example, the reduction of the heat damage to the product and the reduction of the process costs.

The heating, sterilization and cooling steps of the product containers are performed with a recycled rain water. Said water, since it is partially collected in the horizontal cylindrical vessel 2 bottom, it is returned to a temperature by a direct steam injection through parallel pipes 32 located on the bottom of the horizontal cylindrical vessel 2, after it is drawn by the centrifugal pump 31, filtered by the filter 33 and conveyed through the main duct 25 and the pipe 27 to the stationary and movable nozzles 22, 23, respectively. During these steps, the backpressure is kept with air at a temperature equivalent to the one of the process fluid in order to avoid thermal stress conditions in the containers which are the closest to the air flow.

Said filter 33, located in the recirculation water circuit, has the filtering body sized to enable long periods of operation and reduce the number of cleaning operations.

As said before, the water heating occurs rapidly by a direct steam injection through said pipes 32, which have in their longitudinal dimension a series of holes. This system, besides reducing the steam entry speed and uniformly distributing the steam along the horizontal cylindrical vessel 2, substantially reduces the noise of the water impact with respect to the known sterilizers. Obviously, a person skilled in the art in order to met specific and contingent needs, can modify and change the rain water cycle basket rotating sterilizer, however all these changes and modifications are contained in the scope of the invention, as defined in the following claims.

## Claims

1. Rain water (process fluid) cycle rotating drum sterilizer of a type comprising a pressurized horizontal cylindrical vessel (2) receiving rotating baskets (8), containers with a product to be sterilized being housed in the baskets, **characterized in that** it comprises, in combination:
a. stationary nozzles (22), spraying the process fluid, located on the upper part of the horizontal cylindrical vessel (2);
b. nozzles (23) movable with respect to the horizontal cylindrical vessel (2), located on the baskets (8) walls, said nozzles, integrally rotating with said baskets (8), causing water jets to continuously hit a series of predetermined points on the containers with the product;
c. means for adjusting the baskets (8) rotation speed;
d. means for adjusting the flow rate and pressure of the process fluid.

2. Sterilizer (1), according to claim 1, **characterized in that** said stationary nozzles (2) and movable nozzles (23) are supplied by the process fluid collected on the bottom of the horizontal cylindrical vessel (2) by a centrifugal pump (31), the process fluid being filtered by a filter (33) and delivered to the one and the other respectively through a main duct (25) and a pipe (27).

3. Sterilizer (1), according to claims 1 and 2, **characterized in that** said speed adjusting means comprise a ratiomotor (19) driven by an inverter, whose shaft rotates a gear train (20) which, by a pinion (34), transmits the motion to a crown wheel (29) to which a basket holding frame (7) is fixed and consequently to the baskets (8).

4. Sterilizer (1), according to claims 1 and 2, **characterized in that** said means for adjusting the process fluid pressure and flow rate comprise a modulating valve (28) located on the pipe (27), said modulating valve (28) varying said process parameters of each kind of product, product preparation recipe and containers to be sterilized.

5. Sterilizer (1), according to claims 1 and 3, **characterized in that** the baskets (8), receiving the containers with a layered distribution in all the volume and having underlying rollers (30), are inserted and extracted by a chain motor-driven conveyor (9) by means of an automatic engaging and disengaging assembly (10), always in phase, outside driven by a ratiomotor (11) and **in that** said assembly (10) also acts as a centering system having a joint (12), always in phase, made up by conical pins (13) compensating for, at the engagement instant, the position variable of an opposed halfcoupling (14) due to the clearance of the conveyor (9) chain.

6. Sterilizer (1), according to claim 1, **characterized in that** the combination of the baskets (8) rotation, of the vertical water jets coming from the stationary nozzles (22) and of the continuously changing water jets ejected by the movable nozzles (23) generates a turbulence of water which, besides to more easily penetrate the baskets (8) centre, promotes the thermal exchange between the process fluid and the product contained inside the containers.

## Patentansprüche

1. Sterilisator mit Drehtrommel und Regenwasserzyklus (Prozessfluid), umfassend einen druckbeaufschlagten, horizontalen, zylindrischen Behälter (2), der Drehkörbe (8) aufnimmt, wobei Behältnisse mit einem zu sterilisierenden Produkt in den Körben untergebracht sind, **dadurch gekennzeichnet, dass** er Folgendes in Kombination umfasst:
a. ortsfeste, das Prozessfluid sprühende Düsen (22), die im oberen Teil des horizontalen, zylindrischen Behälters (2) liegen;
b. im Bezug auf den horizontalen, zylindrischen Behälter (2) bewegliche Düsen (23), die an den Wänden der Körbe (8) liegen, wobei die Düsen, die sich ganz mit den Körben (8) drehen, dazu führen, dass Wasserstrahlen auf eine Reihe von festgelegten Punkten an den Behältnissen mit dem Produkt treffen;
c. Mittel zum Einstellen der Drehgeschwindigkeit der Körbe (8);
d. Mittel zum Einstellen der Durchflussmenge und des Drucks des Prozessfluids.

2. Sterilisator (1) nach Anspruch 1, **dadurch gekennzeichnet, dass** die ortsfesten Düsen (2) und beweglichen Düsen (23) mit dem Prozessfluid versorgt werden, das am Boden des horizontalen, zylindrischen Behälters (2) durch eine Zentrifugalpumpe (31) gesammelt wird, wobei das Prozessfluid durch einen Filter (33) gefiltert und an die einen und die anderen jeweils durch eine Hauptleitung (25) und ein Rohr (27) geliefert wird.

3. Sterilisator (1) nach den Ansprüchen 1 und 2, **dadurch gekennzeichnet, dass** die geschwindigkeitseinstellenden Mittel einen von einem Inverter angetriebenen Getriebemotor (19) umfassen, dessen Schaft einen Getriebezug (20) dreht, der anhand eines Ritzels (34) die Bewegung an ein Tellerrad (29), an dem ein Korbhalterahmen (7) fixiert ist, und folglich an die Körbe (8) überträgt.

4. Sterilisator (1) nach den Ansprüchen 1 und 2, **dadurch gekennzeichnet, dass** die Mittel zum Einstellen des Drucks und der Durchflussmenge des Prozessfluids ein Modulationsventil (28) umfassen, das auf dem Rohr (27) liegt, wobei das Modulationsventil (28) die Prozessparameter einer jeden Art von Produkt, Produktzubereitungsrezept und zu sterilisierenden Behältnissen variiert.

5. Sterilisator (1) nach den Ansprüchen 1 und 3, **dadurch gekennzeichnet, dass** die Körbe (8), welche die Behältnisse mit einer geschichteten Verteilung im gesamten Volumen aufnehmen und aufweisend darunterliegende Rollen (30), durch einen motorbetriebenen Kettenförderer (9) mithilfe einer automatischen Eingriffs- und Außereingriffseinrichtung (10), die stets phasengleich ist, hinein- und hinausgeschoben werden, die außen durch einen Getriebemotor (11) angetrieben wird, und dass die Einrichtung (10) ferner als ein Zentriersystem wirkt, aufweisend ein Gelenk (12), das stets phasengleich ist, das aus konischen Stiften (13) besteht, die beim Eingriffsmoment die Positionsvariable einer entgegengesetzten Kupplungshälfte (14) aufgrund der Freiräume des Kettenförderers (9) kompensieren.

6. Sterilisator (1) nach Anspruch 1, **dadurch gekennzeichnet, dass** die Kombination der Drehung der Körbe (8), der von den ortsfesten Düsen (22) kommenden vertikalen Wasserstrahlen und der von den beweglichen Düsen (23) ausgegebenen, kontinuierlich wechselnden Wasserstrahlen eine Wasserturbulenz erzeugt, die abgesehen von einer leichteren Durchdringung der Mitte der Körbe (8) den Wärmeaustausch zwischen dem Prozessfluid und dem in den Behältnissen enthaltenen Produkt fördert.

## Revendications

1. Stérilisateur à tambour rotatif à cycle d'eau de pluie (fluide de traitement) d'un type comprenant une cuve cylindrique horizontale pressurisée (2) recevant des paniers rotatifs (8), des récipients avec un produit à stériliser étant logés dans les paniers, **caractérisé en ce qu'**il comprend, en combinaison:
a. des buses immobiles (22), pulvérisant le fluide de traitement, situées dans la partie supérieure de la cuve cylindrique horizontale (2);
b. des buses (23) mobiles par rapport à la cuve cylindrique horizontale (2), situées sur les parois des paniers (8), lesdites buses, étant intégralement en rotation avec lesdits paniers (8), provoquant des jets d'eau afin de frapper en permanence, avec le produit, une série de points prédéterminés sur les récipients;
c. des moyens pour régler la vitesse de rotation des paniers (8);
d. des moyens pour régler le débit et la pression du fluide de traitement.

2. Stérilisateur (1), selon la revendication 1, **caractérisé en ce que** lesdites buses immobiles (2) et buses mobiles (23) sont alimentées par le fluide de traitement recueilli à la base d'une cuve cylindrique horizontale (2) par une pompe centrifuge (31), le fluide de traitement étant filtré par un filtre (33) et amené à chacune d'entre elles par le biais respectivement d'une conduite principale (25) et d'un tuyau (27).

3. Stérilisateur (1), selon les revendications 1 et 2, **caractérisé en ce que** lesdits moyens de réglage de vitesse comprennent un motoréducteur (19) entraîné par un onduleur, dont l'arbre fait tourner un train d'engrenages (20) qui, par un pignon (34), transmet le mouvement à une roue de couronne (29) à laquelle est fixé un cadre de soutien d'un panier (7) et, par conséquent, aux paniers (8).

4. Stérilisateur (1), selon les revendications 1 et 2, **caractérisé en ce que** lesdits moyens de réglage de la pression et du débit du fluide de traitement comprennent une vanne modulante (28) située sur le tuyau (27), ladite vanne modulante (28) modifiant lesdits paramètres de traitement de chaque type de produit, de formule de mélange de produit et de récipients à stériliser.

5. Stérilisateur (1), selon les revendications 1 et 3, **caractérisé en ce que** les paniers (8), recevant les récipients avec une distribution disposée en couche dans tout le volume et disposant de rouleaux sous-jacents (30), sont insérés et extraits par un transporteur à chaîne entraîné par un moteur (9) grâce à un assemblage à embrayage et débrayage automatique (10), toujours en phase, entraîné à l'extérieur par un motoréducteur (11) et **en ce que** ledit assemblage (10) agit aussi comme un dispositif de centrage ayant un raccordement (12), toujours en phase, composé de chevilles coniques (13) compensant, au moment de l'embrayage, la position variable d'un demi-manchon opposé (14) en raison du jeu de la chaîne du transporteur (9).

6. Stérilisateur (1), selon la revendication 1, **caractérisé en ce que** la combinaison de la rotation des paniers (8), des jets d'eau verticaux provenant des buses immobiles (22) et de la modification permanente des jets d'eau éjectés par les buses mobiles (23) génère une turbulence d'eau qui, en plus de pénétrer plus facilement dans le centre des paniers (8), assure l'échange de chaleur entre le fluide de traitement et le produit contenu à l'intérieur des récipients.
